# EUROPEAN PATENT APPLICATION

(11) **EP 2 127 666 A1**
(43) Date of publication of application: **02.12.2009**
(21) Application number: 08157033.5
(22) Date of filing: 28.05.2008
(51) Int. Cl.: A61K 38/17, C07K 14/705, C07K 16/28, C07K 16/42, A61P 37/06

(54) **Method for the treatment or prophylaxis of chronic inflammatory diseases**

(71) Applicant: Drug Discovery Factory B.V., 1404 AK Bussum (NL); Vereniging VU-Windesheim, 1081 HV Amsterdam (NL); Pepscan Therapeutics B.V., 8243 RC Lelystad (NL)
(72) Inventor: Van Egmond, Marjolein, 1081 HV Amsterdam (NL)
(74) Representative: Habets, Winand

(57) **Abstract**

The invention relates to a method for the treatment or prophylaxis of chronic inflammatory diseases. Such diseases may be treated or prevented according to the invention by administering an effective amount of a compound that interferes with binding between IgA and the Fc receptor for IgA ( FcalphaR1 or CD89 ) to a patient in need of such a treatment. In more mechanistic terms, the invention relates to a method for decreasing migration of polymorphonuclear cells and/or infiltration of polymorphonuclear cells by blocking the binding between IgA and CD89. In other terms, the invention relates to a method for preventing activation of polymorphonuclear cells or immune cells by blocking the binding between IgA and CD89, such as by blocking the IgA binding site on CD89 or by blocking the CD89 binding site on IgA.

SEQUENCE LISTING

<110>  Drug Discovery Factory BV
       Vereniging VU-Windesheim
       Pepscan B.V.

<120>  METHOD FOR THE TREATMENT OR PROPHYLAXIS OF CHRONIC INFLAMMATORY
       DISEASES

<130>  040EPP0

<160>  7

<170>  PatentIn version 3.3

<210>  1
<211>  17
<212>  PRT
<213>  Artificial

<220>
<223>  Artificial peptide selected from the Fc region of IgA

<400>  1

Ser Ser Met Val Gly His Glu Ala Leu Pro Leu Ala Phe Thr Gln Lys
1               5                   10                  15

Thr

<210>  2
<211>  18
<212>  PRT
<213>  artificial

<220>
<223>  Artificial peptide: EF loop peptide (CD89 75-92)

<400>  2

Gly Arg Tyr Gln Ala Gln Tyr Arg Ile Gly His Tyr Arg Phe Arg Tyr
1               5                   10                  15

Ser Asp

<210>  3
<211>  11
<212>  PRT
<213>  artificial

<220>
<223>  Artificial peptide; CD loop peptide (CD89 49 - 59)

<400>  3

Glu Ile Gly Arg Arg Leu Lys Phe Trp Asn Glu
1               5                   10

<210>  4
<211>  9
<212>  PRT
<213>  Artificial

<220>
<223>  Artificial peptide

<400>  4

Glu Asp Leu Leu Leu Gly Ser Glu Ala
1               5

<210>  5
<211>  11
<212>  PRT
<213>  Artificial

<220>
<223>  Artificial Peptide

<400>  5

His Glu Ala Leu Pro Leu Ala Phe Thr Gln Lys
1               5                   10


<210>  6
<211>  17
<212>  PRT
<213>  Artificial

<220>
<223>  Artificial peptide

<400>  6

Cys Ser Met Val Gly His Glu Ala Leu Pro Leu Ala Phe Thr Gln Lys
1               5                   10                  15


Cys



<210>  7
<211>  15
<212>  PRT
<213>  Artificial

<220>
<223>  Artificial peptide

<400>  7

Arg Glu Ile Gly Arg Arg Leu Lys Phe Trp Asn Glu Thr Asp Pro
1               5                   10                  15

SEQUENCE LISTING

<110> Drug Discovery Factory BV
Vereniging VU-Windesheim
Pepscan B.V.

<120> METHOD FOR THE TREATMENT OR PROPHYLAXIS OF CHRONIC
INFLAMMATORY DISEASES

<130> 040EPP0

<160> 7

<170> PatentIn version 3.3

<210> 1
<211> 17
<212> PRT
<213> Artificial

<220>
<223> Artificial peptide selected from the Fc region of IgA

<400> 1

Ser Ser Met Val Gly His Glu Ala Leu Pro Leu Ala Phe Thr Gln Lys
1               5                   10                  15

Thr

<210> 2
<211> 18
<212> PRT
<213> artificial

<220>
<223>  Artificial peptide: EF loop peptide (CD89 75-92)

<400>  2

Gly Arg Tyr Gln Ala Gln Tyr Arg Ile Gly His Tyr Arg Phe Arg Tyr
1               5                   10                  15

Ser Asp

<210>  3
<211>  11
<212>  PRT
<213>  artificial

<220>
<223>  Artificial peptide; CD loop peptide (CD89 49 - 59)

<400>  3

Glu Ile Gly Arg Arg Leu Lys Phe Trp Asn Glu
1               5                   10

<210>  4
<211>  9
<212>  PRT
<213>  Artificial

<220>
<223>  Artificial peptide

<400>  4

Glu Asp Leu Leu Leu Gly Ser Glu Ala
1               5

<210>  5
<211>  11
<212>  PRT
<213>  Artificial

<220>
<223>  Artificial Peptide

<400>  5

His Glu Ala Leu Pro Leu Ala Phe Thr Gln Lys
1               5                   10

<210>  6
<211>  17
<212>  PRT
<213>  Artificial

<220>
<223>  Artificial peptide

<400>  6

Cys Ser Met Val Gly His Glu Ala Leu Pro Leu Ala Phe Thr Gln Lys
1               5                   10                  15

Cys

<210>  7

<211>  15

<212>  PRT

<213>  Artificial

<220>

<223>  Artificial peptide

<400>  7

Arg Glu Ile Gly Arg Arg Leu Lys Phe Trp Asn Glu Thr Asp Pro
1               5                   10                  15

## Description

### Field of the invention

The invention relates to a method for the treatment or prophylaxis of chronic inflammatory diseases. Such diseases may be treated or prevented according to the invention by administering an effective amount of a compound that interferes with binding between IgA and the Fc receptor for IgA ( FcalphaR1 or CD89 ) to a patient in need of such a treatment. In more mechanistic terms, the invention relates to a method for decreasing migration of polymorphonuclear cells and/or infiltration of polymorphonuclear cells by blocking the binding between IgA and CD89. In other terms, the invention relates to a method for preventing activation of polymorphonuclear cells or immune cells by blocking the binding between IgA and CD89, such as by blocking the IgA binding site on CD89 or by blocking the CD89 binding site on IgA.

### Background of the invention

Chronic Inflammatory Diseases (CIDs) are an enormous burden on society. Unfortunately adequate and specific treatment is lacking mainly due to the fact that the cause of these diseases remains unknown. CIDs (e.g. Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis) are also an enormous strain on patients and their relatives. Most of these diseases begin early in life and remain a burden for the rest of their lives. CIDs are often debilitating with frequent recurrence making it impossible to work for long periods of time. Furthermore, the psychological effects can be devastating.

Therefore, these diseases are not only associated with considerable morbidity but also have a major impact on the social life of the patient. As a result these conditions also have significant economical consequences.

The available treatments for these diseases tend to be purely symptomatic. They are largely ineffective and non-specific in nature, since the cause remains unknown, despite the enormous effort by the medical society put into studying these diseases. Immunosuppressive drugs can offer temporary relief but the symptoms will almost always re-occur.

Dermatitis herpetiformis is a rare chronic inflammatory skin disorder. The disease is characterized by blistering lesions that are very itchy and tend to concentrate in particular regions of the body. Dermatitis herpetiformis affects approximately 200.000 people in the industrialized countries and can lead to psychosocial problems and a negative self image.

After the diagnosis of Dermatitis herpetiformis has been established a few therapeutic strategies can be considered. The current treatment of the disease is purely symptomatic since the cause of Dermatitis herpetiformis remains largely unknown despite enormous efforts posed by the medical society. The drugs available can offer temporary relief but manifestations of the disease will always reoccur. Hence there is a strong need for the development of a novel treatment for Dermatitis herpetiformis. Therapeutic approaches for Dermatitis herpetiformis include for instance a Gluten-free Diet and treatment with Dapsone, Colchine, Corticosteroids, Sulfapyridine and/or Cyclosporine. These prior art treatments are detailed below.

Approximately 75% of the patients with Dermatitis herpetiformis have symptoms resembling celiac disease. Besides the skin lesions these patients have gastrointestinal problems. Therefore, it is suggested to follow a gluten-free diet once gluten intolerance has been established. The exact response to this therapy is unknown, but up to 50% of the gluten intolerant patients with Dermatitis herpetiformis might benefit from a gluten-free diet. However, it can take more than a decade before an effect is observed. In general, a gluten-free diet takes a lot of discipline. When the effect takes years to be successful the compliance with such an approach is generally low. Although some patients can be free of medication using this treatment, it is in generally not very practical.

Dermatitis herpetiformis is often treated with Dapsone. However, this drug may have unwanted side effects. All patients on Dapsone get a decrease in their haemoglobin levels. This is usually dose-related and is monitored with blood tests. However, some patients get a rapid fall in their blood counts. For this reason, blood tests are performed weekly to begin with and on a monthly basis afterwards. Furthermore, Dapsone can cause headaches, nausea, rash, insomnia, psychosis, peripheral neuropathy, vomiting, sore throat, fever, yellowing of the skin or eyes. Therefore, many patients are reluctant to comply with this treatment.

Colchine is also used in Dermatitis herpetiformis. However, Colchine is not very effective and is associated with side-effects. The most common side-effects involve the stomach and bowel and are dose related. Symptoms include nausea, vomiting, abdominal pain, diarrhoea, hair loss, weakness and nerve irritation. Furthermore, it can cause severe anaemia and low white blood cell counts, which increases the risk of infections. Colchine can arrest cell division and is avoided in pregnancy, because of possible adverse effects on foetal growth.

Corticosteroids (such as prednisone) suppress the immune system and are used to treat moderate to severely active Dermatitis herpetiformis. Although treatment with systemic corticosteroids may control symptoms, the cutaneous manifestations return when they are discontinued. The long-term side effects of the high doses of corticosteroids make this therapy less suitable for maintenance medication.

Sulfapyridine is used as alternative treatment if Dapsone cannot be used. However it is not very effective and has several side-effects.

Some investigational therapies experiment with the use of an elemental diet and systemic cyclosporine. Cyclosporine is a drug that suppresses the immune system, and this may be a promising treatment for people who do not respond to other standard therapies. But this treatment may be associated with a number of side effects, as both unwanted (autoimmunity) as well as wanted (against pathogens) immune responses are suppressed. The latter holds also true for treatment with corticosteroids

Because of the limited number of Dermatitis herpetiformis patients the research and development of new therapeutic approaches within this field is restricted. This is one of the main reasons why new developments in treatment are essentially lacking and alternative treatments are highly desired in the field.

Treatment of patients with Inflammatory Bowel Disease (IBD) such as ulcerative colitis was extensively reviewed by Lichtenstein et al, Gastroenterology 130 (2006), pp. 935-939. The medications reviewed include corticosteroids, azathioprine (AZA), 6-mercaptopurine (6-MP), methotrexate, mycophenolate mofetil, cyclosporine, and infliximab.

According to the Global Strategy for the Diagnosis, Management and Prevention of COPD, Global Initiative for Chronic Obstructive Lung Disease (GOLD) 2007, commonly used treatments in COPD comprise the use of β2 agonists, anticholinergics, methylxanthines, glucocorticosteroids or combinations thereof.

The aim of the present invention is to provide a highly specific and effective drug for the treatment of diseases characterized by infiltration of polymorphonuclear cells, such as chronic inflammatory diseases (CIDs). These diseases include Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis.

### Summary of the invention

It appears that CIDs and in particular Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis share a common mechanism that is responsible for the tissue destruction observed in these diseases. They appear to be characterized by local accumulation of polymorphonuclear cells which are cells of the immune system, more in particular by a local accumulation of neutrophils and/ or eosinophils.

It was hitherto unknown why such accumulation of polymorphonuclear cells occurs. It has now been discovered by the present inventor that binding of IgA to its receptor CD89 triggers a cascade of events including the activation of immune cells that eventually leads to migration, accumulation and infiltration of polymorphonuclear cells.

It is shown herein that IgA has a previously unrecognized role in mediating polymorphonuclear cell migration in the absence of complement, since targeting of polymorphonuclear cell CD89 leads to release of leukotriene B4 (LTB4), which is one of the most potent chemoattractants for polymorphonuclear cells, in particular neutrophils (Parent CA. Curr Opin Cell Biol. 2004;16(1):4-13). Furthermore, as it is herein demonstrated that dlgA, which is normally produced at mucosal surfaces, is capable of mediating polymorphonuclear cell migration as well, we conclude that dlgA plays an active role in maintaining mucosal homeostasis. Whereas SIgA serves as a non-inflammatory antibody at the luminal surface of epithelial cells and constitutes the first line of defense, dIgA functions as an inflammatory antibody at the baso-lateral membrane through interactions with polymorphonuclear cell CD89, which represents a second line of defense.

These discoveries lead to the invention that at least certain CIDs may effectively be treated by interfering with the binding between IgA and its receptor CD89.

The invention therefore relates to a method for decreasing polymorphonuclear cell migration and/or polymorphonuclear cell infiltration by blocking the binding between IgA and CD89.

The invention also relates to a method for decreasing LTB4 excretion from polymorphonuclear cells by blocking the binding between IgA and CD89.

The invention also relates to a method for preventing the activation of immune cells by blocking the binding between IgA and CD89.

The invention also relates to a compound which blocks the binding between IgA and CD89 for the treatment of diseases selected from the group consisting of Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis.

The invention also relates to a compound which blocks the binding between IgA and CD89 for decreasing polymorphonuclear cell migration and/or polymorphonuclear cell infiltration.

The invention also relates to a method for the treatment or prophylaxis of a disease characterized by migration and/or infiltration of polymorphonuclear cells wherein binding between IgA and CD89 is blocked.

The invention also relates to a method for the treatment or prophylaxis of a chronic infectious disease selected from the group consisting of Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis wherein the interaction between IgA and CD89 is blocked.

### Detailed description of the invention

Polymorphonuclear cells or granulocytes are a category of white blood cells characterised by the presence of granules in their cytoplasm. They are also called polymorphonuclear leukocytes. They exhibit a varying shape of the nucleus, which is usually lobed into three segments in the case of neutrophils, and bi-lobed in the case of eosinophils. In common parlance, the term polymorphonuclear cells often refers specifically to neutrophil granulocytes, the most abundant of the granulocytes. Polymorphonuclear cell are released from the bone marrow by granulocyt-colony stimulating factor (G-CSF).

Polymorphonuclear cells are the first line of defense against pathogens within the organism. In particular neutrophils and/ or eosinophils were found to be responsible for the typical extensive tissue damage associated with chronic inflammatory diseases, therefore, these diseases are herein also indicated by the phrase "diseases characterized by migration and/or infiltration of polymorphonuclear cells".

Neutrophil granulocytes, generally referred to as neutrophils, are the most abundant type of white blood cells in humans and form an integral part of the immune system. Their name arrives from staining characteristics on hematoxylin and eosin (H&E) histological or cytological preparations. Whereas basophilic cellular components stain dark blue and eosinophilic components stain bright red, neutrophilic components stain a neutral pink. These phagocytes are normally found in the blood stream. However, during the acute phase of inflammation, particularly as a result of bacterial infection, neutrophils leave the vasculature and migrate toward the site of inflammation in a process called chemotaxis. They are the predominant cells in pus, accounting for its whitish/yellowish appearance. Neutrophils react within an hour of tissue injury and are the hallmark of acute inflammation. Eosinophils are characterized by eosinophilic staining of their granules, and are predominantly involved in parasitic (e.g. worm) infections.

CIDs comprise a family of diseases characterized by infiltration of polymorphonuclear cells. These diseases include Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis.

Whether or not a disease belongs to the group of diseases characterized by infiltration of polymorphonuclear cells may be determined by quantifying granulocyte influx in affected tissue of patients by standard techniques. A particular useful method is exemplified in Example 9.

CD89 is a receptor for the Fc region of IgA and is also known as FCAR or Fc alpha RI. CD89 is encoded by a gene which is a member of the immunoglobulin gene superfamily. The receptor is a transmembrane glycoprotein present on the surface of myeloid lineage cells such as neutrophils, monocytes, macrophages, and eosinophils, where it mediates immunologic responses to pathogens. It binds both IgA isotypes with similar affinity and interacts with IgA-opsonized targets and triggers several immunologic defense processes, including phagocytosis, antibody-dependent cell-mediated cytotoxicity, and stimulation of the release of inflammatory mediators. Ten transcript variants encoding different isoforms have been described for this gene.

CD89 was found to associate with the FcR gamma-chain signaling molecule through a unique charge-based mechanism. CD89 is, thus, connected to the intracellular machinery via the ITAM signaling motifs located within the cytoplasmic tail of FcR gamma-chain. Evidence exists in support of receptors for IgA (distinct from CD89) on human T and B cells. IgA Fc receptors may, therefore, play a role in both the induction and control of an efficient (mucosal) immune response.

IgA is the principal antibody class in mucosal areas (gastrointestinal, respiratory and genito-urinary tracts) as well as in external secretions, and plays a key role in mucosal defense (Woof et al., immunoglobulins.Immunol Rev. 206:64-82). Mucosal surfaces represent a vast interface that shields the interior of the body from external influences. Whereas internal tissues need to remain sterile in order to protect the organism from a potentially life-threatening disease, mucosal surfaces are colonized by commensal microbiota and continuously exposed to inhaled or ingested antigens and pathogens. As such, mucosal homeostasis necessitates a delicate balance between mounting effective immunological responses against pathogenic micro-organisms, while excessive responses against indigenous microflora and dietary/inhaled antigens must be avoided (Pamer EG..Nat Immunol. 2007; 8 (11):1173-8). Interestingly, IgA is involved in both anti- and pro- inflammatory responses (Woof JM, et al.,.J Pathol. 2006;208 (2):270-82., Kerr MA..Biochem J. 1990 ;271 (2):285-96).

At mucosal sides, IgA is produced in the lamina propria by local plasma cells as a dimeric molecule with a joining J- chain, and referred to as dimeric IgA (dIgA) (van Egmond et al. Trends Immunol. 2001 (4):205-11). dIgA is considered as an intermediary molecule, as - after binding to polymeric Ig receptors (pIgR) -, dIgA is transported through epithelial cells and secreted into the lumen as secretory IgA (SIgA) (Brandtzaeg P. Int J Med Microbiol. 2003 293(1):3-15). Although it has been demonstrated that dIgA has the capability to intercept viruses while in transit through infected epithelial cells, and can thus passively interfere with viral replication, an activation role for dIgA has not yet been described (Burns JW, et al., Science. 1996 272 (5258):104-7). SIgA is considered a non- inflammatory mucosal protector. It forms an 'antiseptic' coating of the mucosal wall, which prevents adherence of micro-organisms and neutralizes bacterial products and viruses, and as such serves as first line of defense against invading pathogens (Brandtzaeg P. Int J Med Microbiol. 2003 293(1):3-15.). SIgA is however a poor opsonin and unable to trigger phagocytosis of E. coli bacteria by neutrophilic granulocytes (neutrophils) (van Egmond M et al. Nat Med. 2000 (6):680-5).

Approximately 1-3 mg/ml IgA is furthermore present as monomeric form in blood (serum IgA), where it represents the second prevalent Ab class after IgG. The function of serum IgA is complex and incompletely understood (Woof JM, et al.,.J Pathol. 2006;208 (2):270-82.). On the one hand monomeric serum IgA was demonstrated to display anti-inflammatory activity, and capable of inhibiting IgG- induced inflammatory functions (Nikolova EB et al., Leukoc Biol. 1995 57(6):875-82.).

Additionally, targeting CD89 with monomeric serum IgA results in inhibitory signals or apoptosis in monocytes (Pasquier B et al., Immunity. 2005 (1):31-42, Kanamaru et al.,Blood. 2007;109(1):203-11.). On the other hand, cross- linking of CD89 induces several inflammatory functions such as phagocytosis, antibody-dependent cellular cytotoxicity, antigen presentation and release of cytokines and inflammatory mediators (van Egmond et al. Trends Immunol. 2001 (4):205-11., van Egmond M et al. Nat Med. 2000 (6):680-5. Monteiro RC et al., Annu Rev Immunol. 2003; 21:177-204). The in vivo function for polymorphonuclear cell CD89 has not been elucidated. However, E. coli bacteria that are opsonized with human serum IgA are vigorously phagocytosed by Kupffer cells of CD89 transgenic mice. As such, CD89-serum IgA interactions on Kupffer cells may act as additional line of defense to eliminate bacteria that have invaded the portal circulation (van Egmond M et al. Nat Med. 2000 (6):680-5). Thus, CD89 can act as a bifunctional receptor that induces either anti- or pro-inflammatory functions of IgA, depending on the circumstances (van Egmond M. Blood. 2007; 109:1-2.).

Complement factors can function as chemoattractants for polymorphonuclear cells. In contrast to IgM and IgG, IgA cannot bind C1q, which activates the classical complement cascade. IgA is therefore a poor activator of complement.. Although IgA was shown to activate the alternative or lectin pathways under specific in vitro conditions or in disease (Janoff EN, et al., J Clin Invest. 1999 104(8):1139-47., Roos A, et al.,J Immunol. 2001;167(5):2861-8.), the overall physiological significance of complement activation by IgA is unclear. As such, a role for IgA in mediating polymorphonuclear cell migration has not been identified.

The term "IgA" is herein used to include all species of Immunoglobulin A, including dimeric IgA (dIgA), monomeric IgA, polymeric IgA and secretory IgA (SIgA).

The properties and biological functions of IgA and CD89 in health and disease have been reviewed by Otten and van Egmond in Immunol Lett 2004; 92: 23-31.

The inventor has discovered a common underlying disease mechanism responsible for tissue damage in at least certain CIDs. IgA is normally present in the human body in mucous secretions, including tears, saliva, colostrum, intestinal juice, vaginal fluid and secretions from the prostate and respiratory epithelium. It is also found in small amounts in blood. Because it is resistant to degradation by enzymes, SIgA can survive in harsh environments such as the digestive and respiratory tracts, to provide protection against microbes that multiply in body secretions. It was discovered that IgA interacts with a receptor (CD89) on a specific cell of the immune system, the polymorphonuclear cells, in particular with neutrophils and/ or eosinophils. This interaction induces a cascade of events leading to migration and infiltration of further polymorphonuclear cells to the site where IgA is present and consequently to inflammation and damage of the tissue causing the symptoms of the CID.

CIDs wherein this mechanism plays a particularly important role include diseases such as Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis.

Deposits of IgA are present in the skin of patients with Dermatitis herpetiformis, making this disease a particularly suited model system for investigating the mechanism behind the IgA-induced migration and infiltration of polymorphonuclear cells. We therefore studied the role of IgA in the pathogenesis of Dermatitis herpetiformis as a model system for CIDs wherein IgA is involved.

In Dermatitis herpetiformis, IgA is deposited just below the surface of the skin (dermal papillae), this does not normally occur in healthy individuals. The reason for these IgA deposits is unknown; we herein show that IgA aggregates directly trigger polymorphonuclear cell migration through interaction with a receptor (CD89) on the cell surface of the polymorphonuclear cells. This results in large polymorphonuclear cell accumulations at the site of the IgA depositions. Furthermore, the IgA deposits activate the local polymorphonuclear cells and cause degranulation and release of inflammatory molecules. The polymorphonuclear cell accumulation, activation, and subsequent degranulation are responsible for the damage to the skin and the blister formations. As deposition of IgA is continuous, recruited polymorphonuclear cells will encounter more IgA aggregates. Consequently, these polymorphonuclear cells become activated as well, hereby sustaining a perpetuating inflammatory loop causing tissue destruction.

This finding not only sheds new light on our understanding of CIDs but can directly contribute to the development of a new drug to treat certain CIDs such as Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis. If the interaction between IgA and the receptor on the polymorphonuclear cells such as neutrophils is blocked, then the inflammatory reaction ceases and the patient is cured.

The invention therefore relates to a method for decreasing polymorphonuclear cell migration and/or polymorphonuclear cell infiltration by blocking the binding between IgA and CD89.

The invention also relates to a method for decreasing LTB4 excretion from polymorphonuclear cells by blocking the binding between IgA and CD89.

The invention also relates to a method for preventing the activation of immune cells by blocking the binding between IgA and CD89.

The invention also relates to a compound which blocks the binding between IgA and CD89 for the treatment of diseases selected from the group consisting of Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis.

The invention also relates to a compound which blocks the binding between IgA and CD89 for decreasing polymorphonuclear cell migration and/or polymorphonuclear cell infiltration.

The invention also relates to a method for the treatment or prophylaxis of a disease characterized by infiltration of polymorphonuclear cells wherein binding between IgA and CD89 is blocked.

The invention also relates to a method for the treatment or prophylaxis of a chronic infectious disease selected from the group consisting of Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis wherein the interaction between IgA and CD89 is blocked.

Blocking the interaction between IgA and CD89 or "interfering with" or "interfere with" the binding of IgA to the IgA binding site on CD89, or the CD89 binding site on IgA in this respect means that in effect the binding of IgA to CD89 is decreased. This may mean that less IgA molecules bind to the IgA binding site of the CD89 receptor and/or that IgA binds with less affinity. Decreased binding of IgA to the CD89 receptor may be measured by direct assays as known in the art or as exemplified herein or by functional assays measuring the migration of polymorphonuclear cells to IgA coated beads. (Morton et al., J. Exp. Med. 1999; 189(11):1715-22)

Based on these findings and assisted by the fact that structure of the two molecules involved (IgA and CD89) is known in the art (Herr AB et al. Insights into IgA-mediated immune responses from the crystal structures of human FcalphaRI and its complex with IgA1-Fc.Nature. 2003; 423(6940):614-20), highly specific and effective blocking compounds such as peptides and antibodies can now easily be designed using peptide synthesis techniques known in the art. It is now within the ordinary skills of a person skilled in the art to select the most promising lead candidates using routine laboratory tests.

Suitable molecules that may interact with the binding between IgA and its receptor CD89 are for instance antibodies and peptides, such as synthetic peptides. Antibodies with the desired specificity are commercially available (such as for instance MCA 1824XZ available from AbD Serotec) and have also been described in Zhang et al., Clin. Exp. Immunol 121: 106-11.

Such antibodies are preferably antibodies incapable of complement activation, as described in WO 02/064634. Herein, two classes of anti-CD89 antibodies are distinguished. The first class consists of antibodies not inhibited by IgA, i.e. they bind to CD89 at a different site from the IgA binding site. Also disclosed therein are antibodies that inhibit IgA binding to CD89, i.e. they bind to CD89 at a site which is within or structurally near the IgA binding site. Those latter antibodies may be particularly useful in the present invention.

Moreover, it is suggested in WO 02/064634 that such antibodies may be used for the treatment of diseases characterized by abnormal endogenous IgA. Examples of such diseases are said to be chronic hepatitis, IgA nephropathy or IgA glomerulonephritis. It is clear that such diseases are characterized by circulating complexes of abnormal IgA and/or precipitation of IgA in abnormal IgA immune complexes. The term abnormal is to be interpreted in this context as meaning IgA with a specificity that does not normally occur in the healthy human body and/or IgA complexes which do not normally occur at a certain location at the human body, i.e. at an abnormal location. In chronic hepatitis the abnormal IgA antibody is directed against a viral infection, whereas in IgA nephropathy or IgA glomerulonephritis IgA complexes are found at abnormal locations, i.e. the kidneys.

Furthermore, WO 02/064634 discloses treatment of cancer or autoimmune disorders using bispecific antibodies capable of both targeting the CD89 bearing immune cells and specific target cells.

The present invention describes the use of antibodies or peptides for the treatment of diseases characterized by polymorphonuclear cell migration and/or infiltration, for instance due to the presence of immune complexes of normal IgA at locations where such IgA normally occurs in the healthy human body. The term normal in this context is to be interpreted as meaning IgA with a specificity that does normally occur in the healthy human body and/or IgA complexes which do normally occur at a certain location at the human body, i.e. at a normal location.

WO 02/064634 is silent about decreasing polymorphonuclear cell migration and/or infiltration in such diseases. Examples of diseases for which the present invention provides an effective therapy are for instance Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis.

WO 01/723330 discloses methods and compositions for eliminating pathogens from the circulatory system of a subject. These methods rely on the interaction between monomeric (serum) IgA and CD89 expressed on liver Kupffer cells. The methods employ therapeutic complexes which are made up of a first portion which specifically binds Fc alpha RI expressed on liver Kupffer cells, or which specifically binds monomeric IgA or the Fc region thereof, linked to a second portion which specifically binds the target cell or antigen.

Monteiro et al., describe the generation and isolation of human monoclonal antibodies against CD89. These antibodies were found not to block IgA binding (Monteiro et al., J. Immunol. 148, 1764-1770). Such non-blocking antibodies are described to be useful to achieve inhibition of signal transduction or apoptosis (Kanamura et al., J. Immunol. 2008, 180; 2669-2678).

WO 2005089798 describes the use of a monovalent antibody fragment directed against the EC2 domain of CD89 for the treatment of inflammatory diseases. The EC2 domain is distinct from the IgA binding domain on CD89.

In contrast with the present finding, Sibille et al, disclosed that the chemotaxis of human neutrophils towards chemoattractants may be inhibited by the addition of IgA (Mol. Immunol. (1987) 24; 551-559). At that time, the receptor for IgA was still unknown and it remains unclear whether or not they measured an interaction between IgA and CD89. This is however unlikely since there was no difference observed in the effect of serum IgA and secretory IgA which would be expected in case of CD89.

To investigate whether interfering with the interaction between CD89 and IgA reduces polymorphonuclear cell migration, we performed migration experiments in the presence of anti-CD89 blocking monoclonal antibodies or peptides that mimic either the IgA binding site on CD89 or the CD89 binding site on IgA. When polymorphonuclear cells were pre-incubated with anti-CD89 monoclonal antibodies that specifically interfere with the IgA-binding site on CD89, migration to IgA-beads was reduced.

Antibodies that specifically interfere with the IgA-binding site on CD89 are known in the art. Exemplified herein are antibodies MIP8a, 2D11 or MY43 as described in Morton et al., J. Exp. Med. 1999 Jun 7;189(11):1715-22 and Shen L.A., J Leukoc Biol. 1992 Apr;51 (4):373-8.

It is shown herein that these antibodies are capable of decreasing polymorphonuclear cell migration and/or polymorphonuclear cell infiltration by blocking the binding between IgA and CD89 in that they block the IgA binding site on CD89.

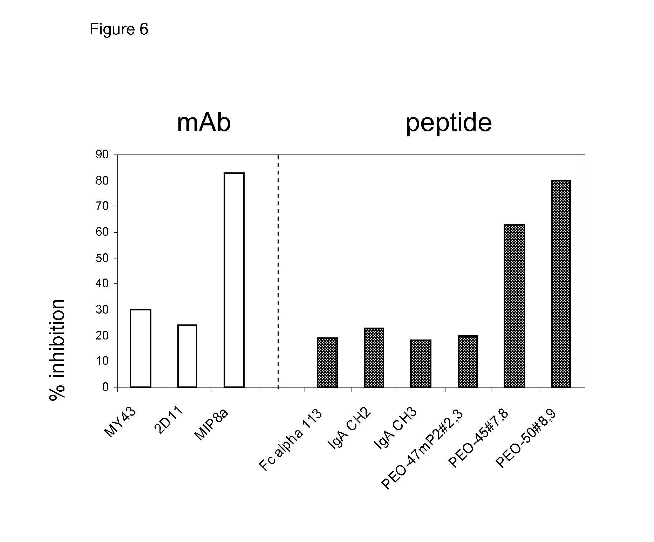

In an experimental set-up as described in Example 4, it was determined that these antibodies were able to effectively block the interaction between IgA and CD89 albeit at different levels. MY43 was able to block the interaction for about 30%, 2D11 showed around 25% blocking whereas Mip8a showed between 75% and 90% blocking (figure 6).

Hence the invention also relates to a compound which blocks the binding between IgA and CD89 for the treatment of diseases selected from the group consisting of Inflammatory Bowel Disease (IBD) such as ulcerative colitis or Crohn's disease, COPD, allergic and non-allergic rhinitis, food allergies such as Celiac disease and skin diseases such as linear IgA bullous disease or Dermatitis herpetiformis.

The invention also relates to a compound which blocks the binding between IgA and CD89 for decreasing polymorphonuclear cell migration and/or polymorphonuclear cell infiltration.

Furthermore, polymorphonuclear cell migration was also reduced when interaction between IgA and CD89s was hindered by addition of peptides that mimic either the IgA binding site on CD89 or the CD89 binding site on IgA. Thus, IgA induces migration of polymorphonuclear cells, which can be reduced by interfering with IgA-CD89 interactions.

The invention therefore also relates to a peptide which blocks the binding between IgA and CD89 for the treatment of diseases selected from the group consisting of Inflammatory Bowel Disease (IBD) such as ulcerative colitis or Crohn's disease, COPD, allergic and non-allergic rhinitis, food allergies such as Celiac disease and skin diseases such as linear IgA bullous disease or Dermatitis herpetiformis.

In other words, the invention relates to a peptide which blocks the binding between IgA and CD89 for decreasing polymorphonuclear cell migration and/or polymorphonuclear cell infiltration.

Specifically designed therapeutic peptides may thus serve the purpose of blocking the specific interaction between IgA and CD89. Peptides have several characteristics making them very attractive as therapeutic agents. Peptides have very specific binding properties to the target of interest and have less accumulation in tissue, reducing the risk of side effects as a result of intermediate metabolite production. They show high specificity ensuring excellent biological activity and low toxicity. This is especially true for drugs with an immuno-modulating effect.

Peptides that interfere with the binding between IgA and the IgA binding site on CD89 may be designed using the known primary structure of either IgA or CD89. The details of the crystal structure of the complex between CD89 and IgA are known and that knowledge greatly facilitates the design of effective peptides. For the design of a synthetic peptide that mimics the surface of the protein interaction site, a region on either IgA or CD89 may be selected that shows the highest amount of binding contacts on a sequential stretch of residues, for instance a stretch with a length of 15 to 25 amino acids. Also, some variations may be made to stabilize the peptides in such a way that they are locked in a biological active structure.

As shown in the examples section, a 17-mer stretch (amino acids 431-447: SSMVGHEAL**PLAF**TQKT (SEQ ID NO: 1) PEO-45#7,8 in figure 6) was selected from the Fc region of IgA and successfully used to interfere with the binding of IgA with the IgA binding site on CD89. Bold and underlined residues were predicted to have most contact in the interaction site. In several different experiments, this peptide consistently showed its ability to block the binding between IgA and CD89 between 55 and 70% when measured in the assay detailed in example 4. Three more peptides were selected from the Fc region of IgG and successfully used to interfere with the binding of IgA with the IgA binding site on CD89. Peptides IgA CH2: EDLLLGSEA (SEQ ID NO: 4), peptide IgA CH3: HEAL**PLAF**TQK (SEQ ID NO: 5), and peptide PEO-47mP2#2,3; CSMVGHEAL**PLAF**TQKC (SEQ ID NO: 6) were found to block the binding between IgA and CD89 for up to 23%, 18% and 20% respectively. This is shown graphically in figure 6.

It is also shown herein that a peptide may successfully be designed using the primary structure of the CD89 receptor as a starting point. It is known that two main CD89 regions contact the Ig interface of IgAFc; they are termed the EF loop and the CD loop, amino acids 75 - 92 and amino acids 49 - 59 respectively.

EF loop peptide (CD89 75-92): GRYQAQY**R**I**GHY**RFRYSD (SEQ ID NO: 2) termed PEO-50#8,9 in figure 6, and CD loop peptide (CD89 49 - 59): EIG**RRLKFW**NE (SEQ ID NO: 3) were found to block the interaction between IgA and CD 89 between 50 and 100%. Peptide Fc alpha 113: REIG**RRLKFW**NETDP SEQ ID NO: 7) blocked the interaction between IgA and CD89 for up to 19% (figure 6).

Since the details of the crystal structure of the CD89:IgA complex are known (Herr et al.), it is now within the routine skills of a skilled person to select further appropriate peptides with the required binding properties.

In conclusion, several peptides were designed that effectively blocked the interaction between IgA and CD89 and hence are useful drug candidates to prevent the migration and/or infiltration of polymorphonuclear cells in diseases such as Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis. As a consequence, these peptides may effectively be used to treat the above diseases.

The drug candidate thus identified may be admixed with a suitable pharmaceutical carrier and tested in the mouse model system as disclosed herein or directly on patients, such as patients with Dermatitis herpetiformis. The drug may in that case be applied locally in an ointment, limited to a few blisters, so that systemic side effects can be avoided. Furthermore, the effectiveness of the drug can be directly monitored and local side effects (rash, itching, etc.) can be observed. This has the additional advantage that phase I clinical trials in healthy volunteers can be omitted.

The potential drug will provide a treatment for CIDs such as Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis but also potentially for other CIDs. Dermatitis herpetiformis is very similar to Ulcerative Colitis, as both diseases are characterized by the presence of normal IgA as well as large polymorphonuclear cell accumulations. Ulcerative Colitis is also a chronic inflammatory disease. Patients with Ulcerative Colitis suffer from abdominal pain, bloody diarrhoea, weight loss and joint pain. These symptoms can be quite severe with frequent relapses. Therefore, this drug might provide a solution for hundreds of thousands of people suffering from Inflammatory Bowel Disease (IBD) such as ulcerative colitis or Crohn's disease, or other CIDs like Chronic Obstructive Lung Disease (COPD), asthma, allergic and non-allergic rhinitis, food allergies such as Celiac disease, and skin diseases such as linear IgA bullous disease or Dermatitis herpetiformis.

The drug could be used to locally target the inflammatory sites using the platform technology of Actogenix (Belgium). In COPD, inhalation therapy could be used whereas treatment systemic diseases will require systemic application.

The content of the articles and publications cited herein are hereby incorporated by reference into the present application.

### Legend to the figures.

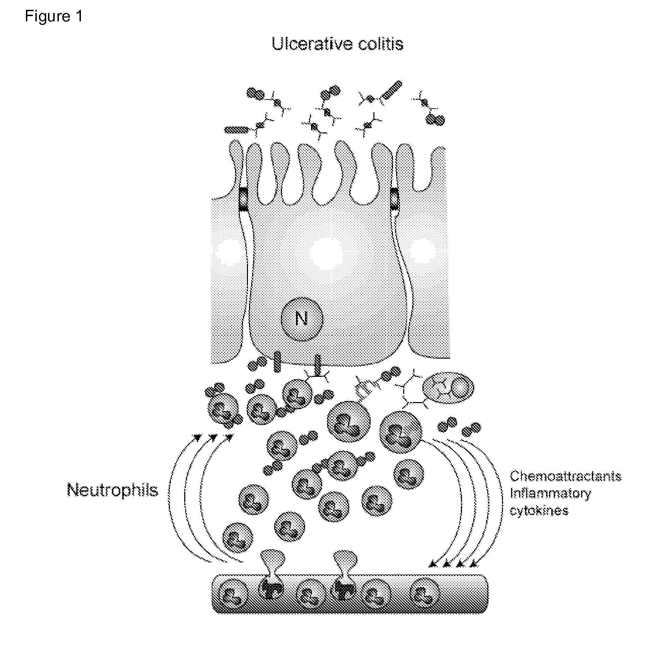

### Figure 1. Inflammatory loop mechanism in Ulcerative Colitis

Role of IgA and CD89 in inflammation of the intestinal tract. When bacteria enter the superficial layers of the gut (lamina propria) they become opsonized with IgA. Polymorphonuclear cells interact with these IgA-opsonized bacteria, and become activated and release molecules that cause migration of immune cells including additional polymorphonuclear cells. Newly arrived polumorphonuclear cells also become activated, which leads to a perpetuating inflammatory loop, resulting in severe tissue damage.

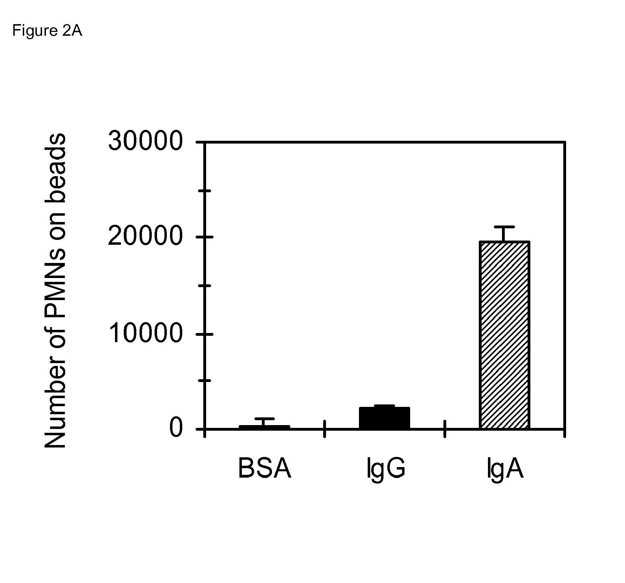

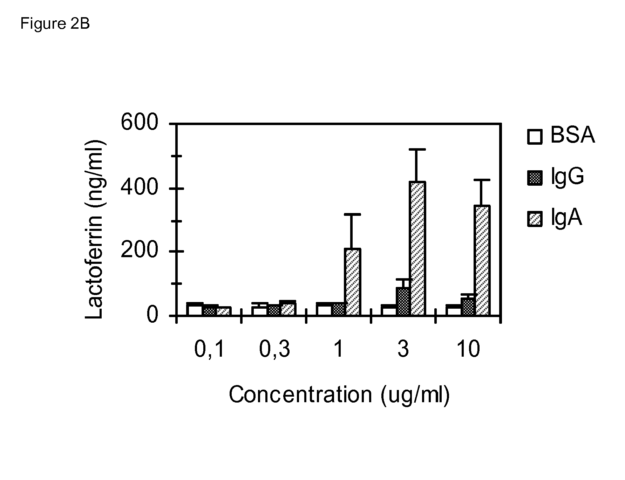

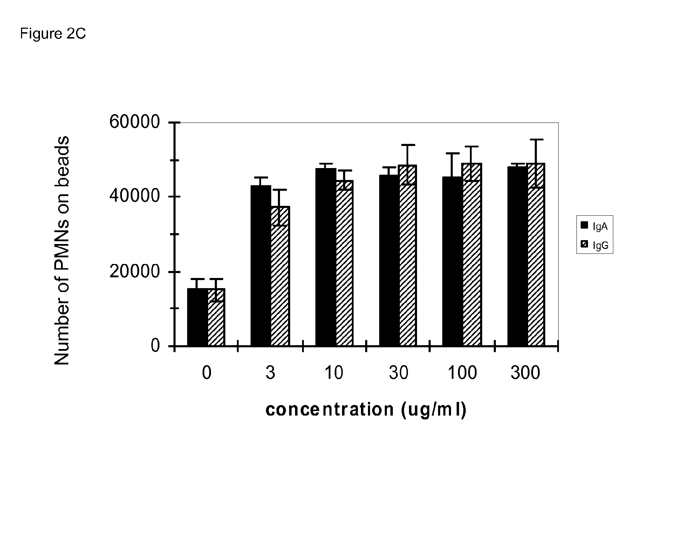

### Figure 2. IgA induces polymorphonuclear cell movement and degranulation.

A) BSA-, IgG- or IgA- coated beads were added to monolayers of resting PKH67-polymorphonuclear cells (PMNs) for 20' at 37°C after which cells were lysed and fluorescence was determined as measure of the number of cells that had migrated to the beads. B) Beads were coated with increasing concentration of BSA, IgG or IgA, and added to monolayers of resting PKH67-polymorphonuclear cells for 20' at 37°C, after which lactoferrin concentration was determined in the supernatant as measure for activation/ degranulation. C) For binding assays IgG- and IgA- coated beads were mixed with PKH67- polymorphonuclear cells for 20'at 4°C, after which cells were lysed and fluorescence was determined as measure of the number of cells that had adhered to the beads. A representative experiment out of six is shown.

### Figure 3. Directed migration of polymorphonuclear cells in response to IgA.

Trajectory paths and direction of polymorphonuclear cells after addition of BSA-, IgG-, or IgA-coated beads were determined (n=30/ situation). Average (A) speed and (B) traveled distance of polymorphonuclear cells in response to coated beads A representative example out of five is shown.

### Figure 4. Cross-linking of polymorphonuclear cell FcαRI by IgA induces release of LTB4.

Supernatants were collected 20' after addition of IgA- , IgG-, or BSA-beads to a monolayer of resting polymorphonuclear cells. A) Numbers of freshly isolated polymorphonuclear cells that have migrated to supernatants in the lower compartments of Blind well chambers. B) Supernatant was collected after addition of IgA-beads, and migration of freshly isolated polymorphonuclear cells to collected supernatant was measured in the presence of anti-BLTR1 (LTB4 receptor), anti-IL-8 or an isotype mAb control. Additionally, either an LTB4 receptor antagonist (U75302 in ethanol) or ethanol alone was added. Migration to supernatant in the absence of inhibitors was set at 100%. c) Presence of LTB4 in supernatants was determined. A representative experiment out of three is shown;.

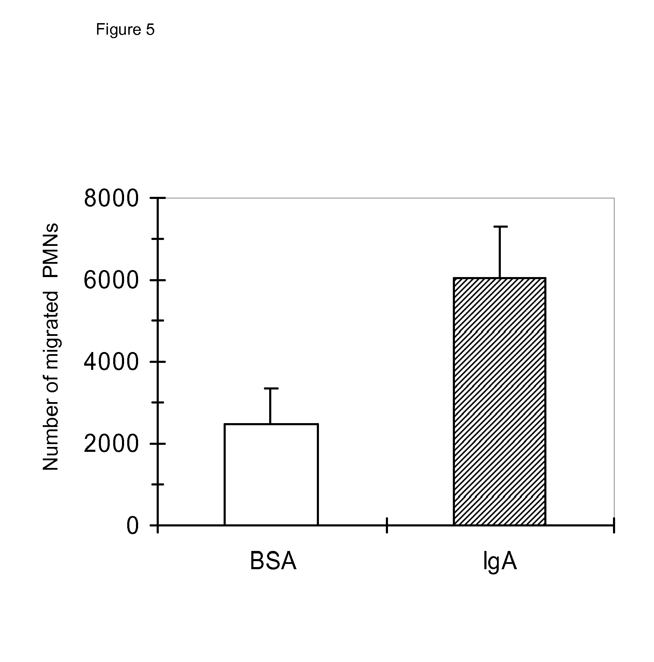

### Figure 5. Phagocytosis of IgA-coated E. coli bacteria leads to release of chemotactic stimuli.

Supernatants were collected 30' after co-incubation of polymorphonuclear cells with either BSA- or IgA-coated E. coli bacteria. Numbers of freshly isolated polymorphonuclear cells that have migrated to supernatants in the lower compartments of Boyden chambers were determined.

### Figure 6. IgA- induced migration can be reduced by interfering with IgA-CD89 interactions.

Percentage inhibition of polymorphonuclear cell migration in the presence of 3 different anti-FcαRI mAb (white bars) or 6 peptides that mimic either the IgA binding site on CD89 or the CD89 binding site on IgA (black bars).

### Examples

### Example 1: Isolation and labeling of polymorphonuclear cells

Polymorphonuclear cells were isolated from heparinized peripheral blood samples, which were obtained from healthy donors, by standard Lymphoprep (Axis-Shield, Oslo, Norway) density gradient centrifugation. To lyse erythrocytes, the pellet was resuspended in an ammonium chloride buffer (155mM, 10', 4°C). Polymorphonuclear cells were resuspended in RPMI 1640 (Gibco BRL, Paisley, UK) supplemented with 10% heat-inactivated fetal calf serum, glutamine and antibiotics (RPMI/10%). Studies were approved by the Medical Ethical Committee of VU University Medical Center (The Netherlands), in accordance with the Declaration of Helsinki. All donors gave informed consent.

Polymorphonuclear cells were fluorescently labeled with PKH67 (PKH67- polymorphonuclear cells), according to the manufacturer's instructions (Sigma-Aldrich, St. Louis, MO). For chemotaxis experiments, polymorphonuclear cells were labeled for 30' at 37°C with 1 µM calcein-AM (Molecular Probes, Eugene, OR). Viability was assessed with trypan blue exclusion. Polymorphonuclear cells were allowed to settle (2h, 37°C) prior to all experiments.

### Example 2: Preparation of immunoglobulin- coated beads.

All experiments were performed with two kinds of immunoglobulin-coated beads. Nitrocellulose beads were made as previously described (Gulle et al., J. Immunol. Methods 214, 199 (1998). Briefly, nitrocellulose (Sigma) was dissolved in dimethylsulfoxide (DMSO) (Sigma) and precipitated by adding milli Q water. Beads of appropriate size were selected using sedimentation times at normal gravity. Pre- wetted nitrocellulose beads were incubated with different concentrations bovine serum albumin (BSA) (Roche Diagnostics, Basel, Switzerland), monomeric IgA (referred to as IgA; Cappel, Solon, OH and Sigma), SIgA (Cappel), IgG (Sigma and Nordic Immunological Laboratories, Tilburg, The Netherlands) for 3h at 4°C. Dimeric IgA (specific for PorA of group B meningococci) was produced as previously described (Vidarsson G et al. J Immunol. 2001; 166:6250-6). Gel separation analyses confirmed that serum IgA was mainly monomeric, whereas SIgA and dimeric IgA consisted of dimers. Beads were centrifuged (20", 14000 rpm), and free protein binding sites were blocked for 30' at 37°C with PBS containing 5% BSA (PBS/BSA). After washing beads were suspended in RPMI/10%. Alternatively, different concentrations of BSA, or immunoglobulins were coupled to CNBr-activated sepharose beads according to the manufacturer's instructions (GE healthcare Bio-Sciences, Uppsala, Sweden).

### Example 3: Three-dimensional Polymorphonuclear cells migration assays:

Collagen gels were prepared as described (Otten et al., J. Immunol. 174: 5472-80 (2005)). Immunoglobulin- coated beads (100µl/ml) were added and 1 ml/ well of this mixture was plated in 24 wells plates and allowed to coagulate, after which 1x10⁶ polymorphonuclear cells were added. After 2h or 4h at 37°C collagen gels were fixed and embedded in paraffin as previously described. Slides were stained with Mayers' hematoxyline (Klinipath, Duiven, The Netherlands).

Real time video recordings of polymorphonuclear cell migration (1x10⁶/ well; 24 wells plate, Greiner Bio One, Kremsmuenster, Austria) were performed with an inverted phase-contrast microscope (Nikon Eclipse TE300, Tokio, Japan) housed in a humidified, 5% CO₂ gassed, temperature-controlled (37°C) chamber. Randomly selected fields were recorded for 20' min. Pictures were taken every 15" with an Olympus ColorView II camera. For tracking experiments an interval of 7" was used. Recordings were analyzed using CELL F trackIT software (Olympus Soft Imaging Solutions GmbH, Münster, Germany).

### Example 4: Two-dimensional Polymorphonuclear cells migration assays

PKH67- polymorphonuclear cells (2.5 x 10⁵ cells/ well) were seeded in 96 wells flat bottom plates (Greiner), after which immunoglobulin- coated beads (10 µl/ well) were added. For binding assays, cells were kept at 4°C. For migration assays, cells were incubated for 20' 37°C, after which cell suspensions were collected and beads were washed 2 times with PBS to remove unbound PKH67- polymorphonuclear cells. Beads were incubated with a buffer containing 2.0 g/I hexadecyltrimethyl ammonium bromide, 1.0 g/I tween 20, 2.0 g/I BSA and 7.44 g/I EDTA in PBS to lyse bound PKH67-polymorphonuclear cells. Fluorescence was measured using 485 excitation and 520 emission filters (Fluostar Galaxy, BMG Labtechnologies, Offenburg, Germany).

Furthermore, PKH67- polymorphonuclear cells (1x10⁶/ well) were added to 24 wells flat bottom plates containing a round MatrigeITM-coated glass cover slip (1:4 diluted in PBS; BD Biosciences, Franklin Lakes, NJ). Immunoglobulin- coated beads (40 µl/ well) were added, and after a 20' incubation time at 37°C, plates were centrifuged (5', 500 rpm). Cells were fixed with 4% buffered formaline in PBS (20', RT). Cover slips were embedded with Aquamount (Gurr BDH Chemicals Ltd, Poole, UK) and analyzed with a Leica DM6000B fluorescence microscope (Leica Microsystems, Heidelberg, Germany) and a Leica TCS SP2 AOBS Confocal laser-scanning microscope.

### Example 5: Animal experiments.

Generation of FcαRI transgenic (Tg) mice was described earlier (van Egmond et al., Blood 93: 4387-94 (1999)). Mice were bred and maintained at the Central Animal Facility of the VU University Medical Center, Amsterdam, The Netherlands under standard conditions. BSA- or IgA-coated beads were injected intradermally (30µl) in PBS/ Indian ink 1:100 to visualize injection sites. Animals were sacrificed after 72h and skin biopsies were taken. Polymorphonuclear cells presence was visualized by staining slides with rat anti-mouse GR-1 mAb (BD Biosciences), and horseradish peroxidase (HRP)-labeled goat anti-rat antibodies. 3-Amino-9-ethylcarbazole (BD Biosciences) was used as substrate. All experiments were performed according to institutional and national guidelines.

### Example 6: Chemotaxis assay

Chemotaxis assays were essentially performed as described (Frevert et al., J. Immunol. Methods 213, 41 (1998)), modified for use in a 48 well Neuroprobe blind well chemotaxis chamber (Gaithersburg, MD). Briefly, bottom wells were filled with supernatants of migration experiments (25 µl), and covered with a 3 µm pore polycarbonate filter (Neuroprobe). Purified LTB4 (Sigma) or IL-8 (Peprotech, Rocky Hill, NJ) were used as positive controls. Top wells were filled with 50 µl calcein AM- labeled polymorphonuclear cells (1x10⁶/ml). After a 40 minutes incubation period at 37°C, cells that had migrated towards the lower compartments were quantified with a fluorimeter (485 excitation/ 520 emission filters). To inhibit IL-8 mediated migration, experiments were conducted in the presence of 100 µg/ml blocking anti-IL-8 mAb (BD Biosciences). LTB4-mediated migration was investigated by blocking LTB4 receptors with 100 µg/ml anti-BLTR1 mAb (BD Biosciences). Additionally, the LTB4 receptor antagonist U75302 (10µM in ethanol; Sigma) was added during chemotaxis experiments.

### Example 7: Enzyme-Linked ImmunoSorbent Assays

LTB4 in supernatants was determined using an ELISA kit from R&D systems (Minneapolis, MN), whereas IL-8 was determined using matched antibody pairs from Biosource (Nivelle, Belgium). For the determination of lactoferrin, rabbit anti-human lactoferrin (Sigma) was used as catching antibody, alkaline phosphatase-labeled anti-human lactoferrin (MP Biomedicals, Eindhoven) as detecting antibody and p-nitrophenyl phosphate as chromogenic substrate.

### Example 8: Phagocytosis assay

Polymorphonuclear cells were mixed with E. coli bacteria (F1 strain, overnight culture in liquid broth [Oxoid, Cambridge UK]; E:T ration 1:10) in the presence of BSA or IgA (1 mg/ml), and incubated at 37°C on a rotatory shaker (8 rpm) for 30'. Cells were centrifuged for 8' at 150*g). Supernatant was collected and centrifuged for 10' at 15.000*g, and stored for further analysis in chemotaxis assays. Cells were washed twice, and cytospin preparations were made.

### Example 9: Quantification of polymorphonuclear cell influx in tissues of patients.

Biopsies may be taken from inflamed tissue of patients in order to determine influx of granulocytes. These biopsies may then be processed for histological or immunohistochemical analyses as known in the art. Briefly, biopsies are either snap-frozen or fixated overnight in 10% phosphate-buffered formalin (4% paraformaldehyde), dehydrated in increasing concentrations of ethanol, and embedded in paraffin. Sections are then cut and stained with haematoxylin and eosin (H&E) for histological analysis. Sections are either stained with monoclonal antibody EG2 (Phadia, dilution 1:200) which detects the eosinophil granule protein ECP, or with a monoclonal antibody against the neutrophil granule protein MPO (Dako, A390, dilution 1:8000) following a standard immunohistochemical protocol (Widegren et al., Respir Res. 2008;9:15). The numbers of ECP-positive (eosinophils) and MPO-positive (neutrophils) in the tissues are counted, and compared to the number of granulocytes in tissues of healthy donors. Randomly taken pictures of tissues were captured using a camera at 10x in a standard procedure with fixed exposure time for all photos as described (Floris S, et al. Brain 2004;127:616-627).The polymorphonuclear cell influx is quantified by counting individual cells. Accumulated clusters of cells will be quantified with the use of the digital image analysis program AnalySIS (Soft Imaging System GmbH, Münster, Germany). By means of a constant predefined threshold for color components, positively stained areas are used for quantification. It is concluded that a disease is characterized by infiltration of polymorphonuclear cells if an increased number of polymorphonuclear cells is detected compared to the number of cells in corresponding tissues of healthy donors.

Alternatively, in patients with lung inflammation, the cell number may be determined in a bronchoalveolar lavage (BAL) sample that may be taken during routine diagnostic bronchoscopy (Babusyte A et al. Respir Res. 2007 Nov 14;8:81).. Briefly, the bronchoscope (Olympus, USA) is wedged into the segmental bronchus of the middle lobe and 20 mL × 7, (total 140) mL of sterile saline solution (0.9% NaCl) is infused. Fluid is filtered using 40 µm cell stainer (Becton Dickinson, USA) and centrifuged at 4°C for 10 min. Total cell numbers are determined using the Trypan blue exclusion method, after which cytospin preparations are made. BAL cytospins are stained by the standard May-Grünwald-Giemsa method for differential cell counts. Cell differentiation is determined by counting 200 cells in random fields of view under light microscope. The cells are identified using standard morphological criteria, by nuclear morphology and cytoplasmic granulation or by immunohistochemical stainings as described above It is concluded that lung inflammation is characterized by infiltration of polymorphonuclear cells if an increased number of polymorphonuclear cells is detected compared to the number of cells in corresponding BAL of healthy donors.

### Example 10: IgA induces activation and directed migration of polymorphonuclear cells.

Migration of polymorphonuclear cells towards IgA- coated Sepharose beads was observed after 2 hours in a 3 dimensional culture assay developed for this purpose and detailed in Example 3. However, migration towards bovine serum albumine (BSA)- or IgG- coated beads was neither observed after 2 hours, nor after later time points. In addition, in a second 3 dimensional culture migration assay, migration to IgA-, but not to BSA- or IgG- coated nitrocellulose beads was observed. Thus, IgA specifically induces migration of polymorphonuclear cells.

Next, real time video recording assays were performed to visualize polymorphonuclear cell migration towards immunoglobulin-coated targets in more detail. In these experiments, polymorphonuclear cells were labeled with the fluorescent dye PKH67. All experiments were conducted with both Sepharose and nitrocellulose beads, which yielded comparable results. Therefore, we refer to IgG- or IgA-beads (or BSA-beads as control) hereafter without distinguishing between Sepharose and nitrocellulose. Polymorphonuclear cells did not respond to BSA-beads or IgG-beads, and polymorphonuclear cell were still inactive after 20 minutes, indicated by their round shape. In contrast, when IgA-beads were added, cells in close proximity of adherent polymorphonuclear cells became activated within 5 minutes (reflected by their elongated cell bodies), after which the activation front spread towards neighboring cells. The amount of bound cells was quantified by measuring fluorescence after the cells had been lysed, which confirmed that only few polymorphonuclear cells had bound to BSA- or IgG-beads, whereas a large number of polymorphonuclear cells had migrated towards and adhered to IgA-beads (Fig. 2A). Similar results were observed with different immunoglobulin concentrations or after longer time points. In addition, polymorphonuclear cells degranulated after binding to IgA-beads, reflected by lactoferrin release (as component of secondary granules), but not after binding to IgG or BSA-beads, hereby indicating that polymorphonuclear cells became only activated after binding to IgA-beads (Fig. 2B). No difference in polymorphonuclear cell binding capacity to IgA- or IgG-beads was observed, which excluded the possibility that differences in IgA- versus IgG-initiated activation and migration were due to poorer adherence of polymorphonuclear cells to IgG-beads (Fig. 2C).

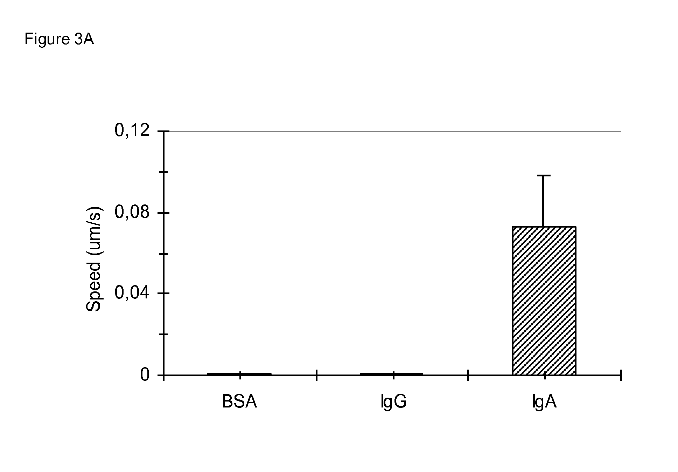

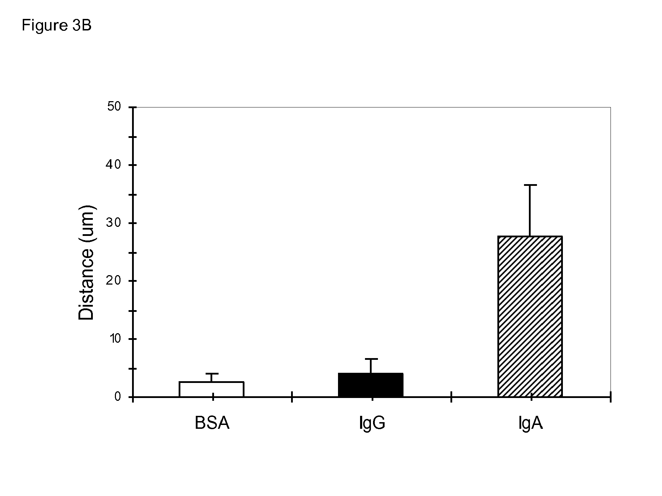

To confirm that cell movement was specifically directed towards IgA-beads, and not merely the result of increased random chemokinesis, cell tracking experiments were performed. The position of cells was determined every 7 seconds for 20 minutes. In response to BSA- or IgG-coated beads polymorphonuclear cells did not gain any speed and covered only minimal random distance. However, in the presence of IgA-beads polymorphonuclear cells rapidly increased speed after activation , and traveled in the direction of the IgA-beads (Fig. 3). Thus, IgA induces directed migration of polymorphonuclear cells.

### Example 11: In vivo IqA- induced polymorphonuclear cell migration is mediated through interaction with CD89 .

In vivo studies investigating the role of IgA have been restricted, since mice do not express an CD89 homologue (van Egmond et al. Trends Immunol. 2001 (4):205-11. Monteiro RC et al., Annu Rev Immunol. 2003; 21:177-204.). To overcome this limitation we previously created human CD89 transgenic (Tg) mice, in which CD89 expression pattern, regulation, interaction with human IgA, and function mimic the human situation (van Egmond M, et al.,Blood. 1999 93(12):4387-94.). However, since human CD89 does not interact very well with murine IgA we injected BSA- or human IgA beads in the skin of CD89 Tg mice or non-Tg littermates to investigate whether human IgA induces polymorphonuclear cell migration in vivo, and whether this process is dependent on CD89. Massive polymorphonuclear cell accumulation was observed in the skin of CD89 Tg mice, but not in the skin of non-Tg littermates after injection of IgA-beads. Only few polymorphonuclear cells were observed after injection of BSA-beads. Thus, IgA induces polymorphonuclear cell migration in vivo, which is dependent on CD89.

### Example 12: Interaction of IgA with CD89 on polymorphonuclear cells triggers release of LTB4.

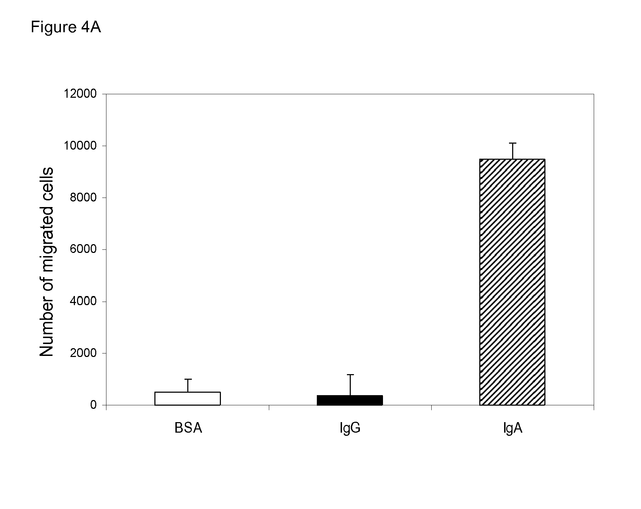

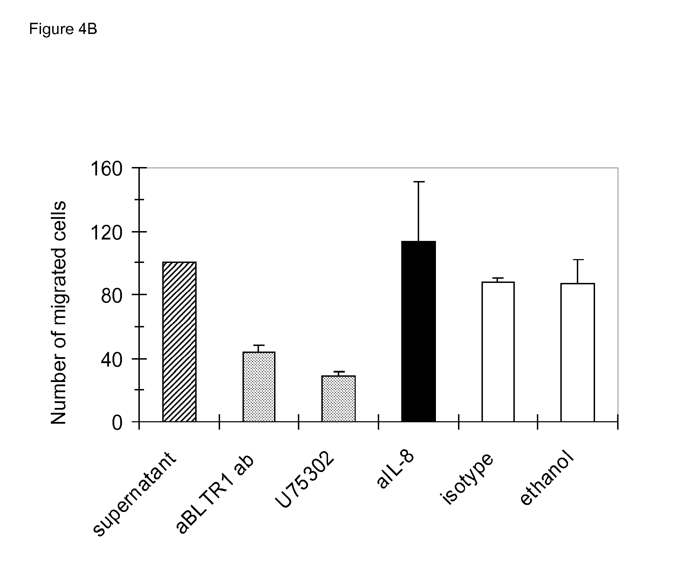

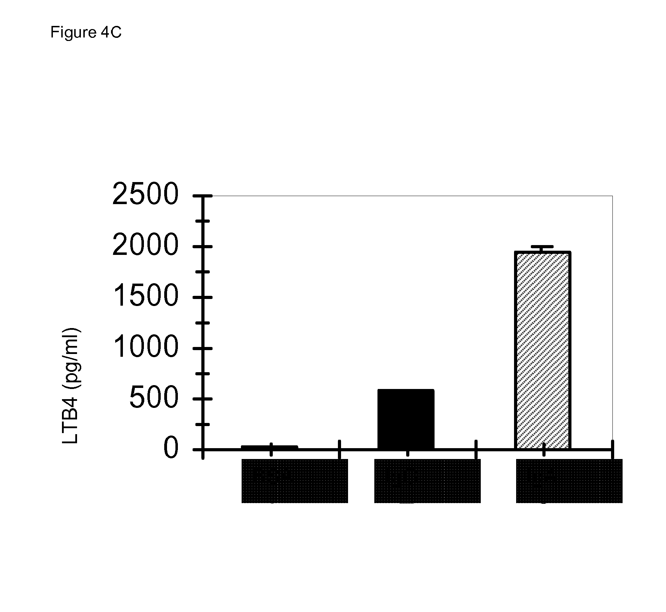

Directed migration of polymorphonuclear cells in response to IgA-beads suggested the release of a chemotactic stimulus, and supernatants of migration experiments were therefore tested in a chemotaxis assay. Only supernatants of experiments in which polymorphonuclear cells had migrated towards IgA-beads, but not to BSA- or IgG-beads showed chemotactic activity (Fig. 4A), which was reduced when LTB4 receptors were blocked by addition of either a blocking anti- BLTR1 mAb or a receptor antagonist (Fig. 4B). Addition of a blocking anti-interleukin 8 (IL-8) mAb did not decrease chemotactic activity. Additionally, minimal amounts of IL-8, but ample amounts of LTB4 were detected in supernatants of migration experiments (Fig. 4C), hereby indicating that IgA induces release of LTB4, which is responsible for the observed polymorphonuclear cell migration.

### Example 13: IqA-induced phagocytosis of E. coli bacteria triggers polymorphonuclear cell migration

To mimic a more physiological situation, we investigated the migratory response after phagocytosis of E. coli bacteria, which are amongst the most common inhabitants of the gut flora. Only minimal phagocytosis of BSA-coated E.coli bacteria by polymorphonuclear cells was observed. However, IgA- coated E. coli bacteria were vigorously phagocytosed. Since chemotactic activity was increased in supernatants, phagocytosis of IgA-coated bacteria resulted in release of chemotactic stimuli (Fig. 5). Because IgA is not produced as monomeric molecule at mucosal sites, but as dimeric molecule, we investigated the ability of dlgA to induce polymorphonuclear cell migration as well. No difference between capacity of inducing polymorphonuclear cell migration was observed between monomeric serum IgA or dIgA.Thus, phagocytosis of IgA-coated bacteria results in polymorphonuclear cell migration, and both monomeric and dimeric IgA are equally capable of inducing polymorphonuclear cell migration.

### Example 14: Peptide synthesis

Peptides were synthesized by solid-phase peptide synthesis using a 4-(2_,4_₋dimethoxyphenyl-Fmoc-aminomethyl)-phenoxy (Rink-Amide) resin (BACH EM, Germany) on a Syro-synthesizer (MultiSynTech, Germany). All amino acids were purchased from BACHEM Biochemica GmbH (Heidelberg, Germany) and used as N-α-(Fmoc) protected with side-chain functionalities protected as N-t-Boc (KW), O-t-Bu (DESTY), N-Trt(HNQ), S-Trt (C), S-StBu (C), or N-Pbf (R) groups. A coupling protocol using a 6.5-fold excess of HBTU/HOBt/amino acid/DIPEA (1:1:1:2) in NMP with a 30 min activation time using double couplings was employed. Peptides were cleaved from the resin by reaction with TFA (15 ml g⁻¹ resin) containing 13.3% (w) phenol, 5% (v) thioanisole, 2.5%(v) 1,2-ethanedithiol, and 5%(v)milliQ-H2O for 2-4 h at RT. The crude peptides were purified by reversed-phase high performance liquid chromatography (RPC), either on a 'Delta Pack' (25 or 40×100 mm inner diameter, 15 µm particle size, 100 A pore size; Waters, U.S.A.) or on a 'XTERRA' (50×4.6 mm inner diameter, 2.5 µm particle size (Waters, U.S.A.) RP-18 preparative C18 column with a lineair AB gradient of 1-2% B min⁻¹. where solvent A was 0.05% TFA in water and solvent B was 0.05% TFA in ACN. The correct primary ion molecular weights of the peptides was confirmed by electron-spray ionization mass spectrometry on a Micromass ZQ (Micromass, The Netherlands) or a VG Quattro II (VG Organic, U.K.) mass spectrometer.

### Example 15 Blocking assays

The blocking potential of anti-FcαRI antibodies and/ or peptides that mimic either the IgA binding site on CD89 or the CD89 binding site on IgA was investigated using a 2-dimensional polymorphonuclear cells migration assay as detailed in example 4. For that purpose, PKH67- polymorphonuclear cells were incubated with different concentrations (ranging from 1-100 µg/ml in RPMI) of either anti-FcaRI blocking antibodies or with peptides mimicking the IgA binding site on CD89 for 20 minutes at 37°C. IgA- coated beads were incubated with different concentrations (ranging from 1-100 µg/ml in RPMI) of peptides mimicking the CD89 binding site on IgA for 20 minutes at 37°C. PKH67- polymorphonuclear cells (2.5 x 10⁵ cells/ well) that had not been incubated (as control) or PKH67- polymorphonuclear cells that had been incubated with blocking antibodies or peptides were seeded in 96 wells flat bottom plates (Greiner). Either non-incubated IgA-coated beads, or IgA-coated beads that had been incubated with peptides were added (10 µl/ well). Cells were then incubated for 20' at 37°C, after which cell suspensions were collected and beads were washed 2 times with PBS to remove unbound PKH67- polymorphonuclear cells. Beads were incubated with a buffer containing 2.0 g/I hexadecyltrimethyl ammonium bromide, 1.0 g/I Tween 20, 2.0 g/I BSA and 7.44 g/I EDTA in PBS to lyse bound PKH67- polymorphonuclear cells. Fluorescence was measured using 485 excitation and 520 emission filters (Fluostar Galaxy, BMG Labtechnologies, Offenburg, Germany) to determine the number of polymorphonuclear cells that had bound to IgA-coated beads. Percentage blocking was determined using the following equation: 100 - (number of bound cells in blocking situation/ number of bound cells without blocking x 100)%.

## Claims

1. Method for decreasing polymorphonuclear cell migration and/or polymorphonuclear cell infiltration by blocking the binding between IgA and CD89.

2. Method for decreasing LTB4 excretion from polymorphonuclear cells by blocking the binding between IgA and CD89.

3. Method for preventing the activation of immune cells by blocking the binding between IgA and CD89.

4. Method according to any one of claims 1 to 3 wherein the IgA binding site on CD89 is blocked.

5. Method according to any one of claims 1 to 3 wherein the CD89 binding site on IgA is blocked.

6. Method according to any one of claims 1 to 5 wherein an antibody is used for blocking.

7. Method according to any one of claims 1 to 5 wherein a peptide, preferably a synthetic peptide, is used for blocking.

8. Compound which blocks the binding between IgA and CD89 for the treatment of diseases selected from the group consisting of Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis.

9. Compound which blocks the binding between IgA and CD89 for decreasing polymorphonuclear cell migration and/or polymorphonuclear cell infiltration.

10. Compound according to claims 8 or 9 capable of blocking the IgA binding site on CD89.

11. Compound according to claims 8 or 9 capable of blocking the CD89 binding site on IgA.

12. Compound according to any one of claims 8 to 11 which is an antibody.

13. Compound according to any one of claims 8 to 11 which is a peptide, preferably a synthetic peptide.

14. Method for the treatment or prophylaxis of a disease **characterized by** infiltration of polymorphonuclear cells wherein binding between IgA and CD89 is blocked.

15. Method for the treatment or prophylaxis of a chronic inflammatory disease selected from the group consisting of Inflammatory Bowel Disease (IBD) such as ulcerative colitis or Crohn's disease, Chronic Obstructive Lung Disease (COPD), asthma, allergic and non-allergic rhinitis, food allergies such as Celiac disease, and skin diseases such as linear IgA bullous disease or Dermatitis herpetiformis wherein the interaction between IgA and CD89 is blocked.
